# EUROPÄISCHE PATENTSCHRIFT

(11) **EP 1 187 823 B1**
(45) Veröffentlichungstag und Bekanntmachung des Hinweises auf die Patenterteilung: **20.08.2003**
(21) Anmeldenummer: 00940322.1
(22) Anmeldetag: 06.06.2000
(51) Int. Cl.: C07D 311/60, C07D 311/30

(54) **GEWINNUNG VON ISOQUERCETIN AUS BIOFLAVANOIDPASTEN**
RECOVERY OF ISOQUERCETIN FROM BIOFLAVANOID PASTES
OBTENTION D'ISOQUERCETINE A PARTIR DE PATES BIOFLAVONOIDIQUES

(30) Priorität: 16.06.1999 DE 19927425
(43) Veröffentlichungstag der Anmeldung: 20.03.2002
(73) Patentinhaber: MERCK PATENT GmbH, 64293 Darmstadt (DE)
(72) Erfinder: BUCHHOLZ, Herwig, D-60599 Frankfurt (DE); JUNGNITZ, Michael, D-60385 Frankfurt (DE); GRUND, Michael, D-64293 Darmstadt (DE); ROSSKOPF, Ralf, D-64839 Münster (DE); HÄRTNER, Hartmut, D-64367 Mühltal (DE)
(86) Internationale Anmeldenummer: EP0005182
(87) Internationale Veröffentlichungsnummer: WO00076992

(56) Entgegenhaltungen:
- WO-A-00/26399
- CHEMICAL ABSTRACTS, vol. 67, no. 17, 23. Oktober 1967 (1967-10-23) Columbus, Ohio, US; abstract no. 82035k, Seite 7729; XP002149959 & M. KROLIKOWSKA: ROCZ. CHEM., Bd. 41, Nr. 3, 1967, Seiten 529-40,

## Beschreibung

Die vorliegende Erfindung betrifft ein Verfahren zur Gewinnung von hochreinem Isoquercetin aus Bioflavanoid-Mutterlaugenrückständen, durch Extraktion dieser Rückstände mit einem Lösungsmittelgemisch aus Methylacetat und Wasser.

Isoquercetin und auch andere Flavanoide, wie z. B. Rutin, werden seit kurzem intensiv erforscht wegen ihrer besonderen Eigenschaften als Antioxidantien. Freie Radikale und reaktive Sauerstoff-Verbindungen spielen im Körper des Menschen eine große Rolle bezüglich des Alterns und der Entstehung von Krankheiten.

Antioxidantien wie die Flavanoide oder auch Vitamin C können solche Radikale und Substanzen abfangen und somit deren Wirkung auf den Organismus vermindern.

Flavanoide, insbesondere das Isoquercetin, welches auch in Kombination mit Vitamin C eine große Rolle spielt, werden daher heutzutage oft in pharmazeutischen Formulierungen als Lebensmittelzusatzstoffe verwendet und verabreicht.

Daher steigt der Bedarf an hochreinen Flavanoiden wie Quercetin, Rutin oder insbesondere Isoquercetin. Die Verfahren sollen kostengünstig sein, und die hochreinen Produkte sollen in möglichst wenig Reaktionsstufen erhalten werden.

Es bestand daher die Aufgabe, ein effektives Verfahren zur Gewinnung von hochreinem Isoquercetin aus einem möglichst billigen Ausgansmaterial zu finden, wobei die Reinheit des Isoquercetins vorzugsweise höher als 90 % (HPLC) sein soll.

Überraschenderweise wurde nun gefunden, dass man hochreines Isoquercetin aus Bioflavanoidpasten gewinnen kann durch Extraktion dieser Rückstände mit einem Lösungsmittelgemisch aus Methylacetat und Wasser.

Gegenstand der Erfindung ist daher ein Verfahren zur Gewinnung von Isoquercetin aus Bioflavanoidpasten (Mutterlaugenrückstände, die bei der Rutinherstellung aus Dimorphandra Gardneriana anfallen z.B.), welches dadurch gekennzeichnet ist, dass folgende Schritte durchgeführt werden:
a) Extrahieren der Mutterlaugenrückstände (Pasten) mit einem Lösungsmittelgemisch aus Methylacetat und Wasser im Volumenverhältnis 9:1 in zehnfachem Überschuss, bezogen auf die Masse der eingesetzten Mutterlaugenrückstände, und zwar bei einer Extraktionstemperatur von 35° bis 50°C, und einer Extraktionszeit von mindestens 30 Minuten, und Abschleudern vom festen Raffinat,
b) Verdünnen des Extraktes mit Wasser,
c) Abdestillieren des Methylacetats aus dem Extrakt unter Normaldruck,
d) rasches Abkühlen des wässrigen Destillationsrückstandes auf 10°C, wobei das Isoquercetin auszufallen beginnt,
e) umgehendes Abtrennen und Trocknen des Produktes.

Die Bioflavanoidpasten sind Mutterlaugenrückstände, die bei der Herstellung von reinen Flavanoiden zurückbleiben, und die meistens noch weitere Flavanoide in größerer Menge enthalten. Diese Rückstände stellen ein günstiges Ausgangsmaterial dar für die Gewinnung anderer Flavanoide dar.

Beispielsweise enthält Dimorphandra Gardneriana (Fava d'anta) neben Rutin als zweites Flavanoid Isoquercetin, das bei der Rutinherstellung zwangsläufig dann in der Mutterlauge mit anfällt. Die Extraktion solcher Mutterlaugenrückständen (Pasten) zur Gewinnung von Isoquercetin ist Gegenstand dieser Erfindung.

Das Isoquercetin wird aus den Mutterlaugenrückständen ('Pasta Seca') mit einem Lösungsmittelgemisch von Methylacetat und Wasser im Volumenverhältnis 9:1 im zehnfachen Überschuß, bezogen auf die Masse der eingesetzten Mutterlaugenrückstände, d.h. 10 I Extraktionsmittel auf 1 kg Pasta Seca, extrahiert und vom festen Raffinat abgeschleudert. Das Raffinat läßt sich, aufgefüttert mit frischer Pasta Seca, zu weiteren Extraktionen einsetzen.

Der Rohstoff wird dabei vorzugsweise vorgesiebt und langsam in den Reaktor eingetragen.

Vorzugsweise wird säurefreies Methylacetat verwendet, damit eine mögliche Hydrolyse des Isoquercetins unterbunden wird. Daher wird das Lösungsmittelgemisch gepuffert, beispielsweise mit einem Puffer wie Ammoniumhydrogencarbonat.

Die Extraktionstemperatur liegt bei 35° bis 50°C, und damit deutlich unter dem Siedepunkt von Methylacetat (55° bis 57°C). Bei höherer Temperatur hat man mit Ausbeuteverlusten, Extraktionsmittelverlusten und Filtrationsschwierigkeiten zu rechnen.

Die Extraktionszeit beträgt mindestens 30 Minuten. Vorzugsweise wird 30 bis 60 Minuten lang extrahiert. Eine längere Extraktionszeit von bis zu 6 Stunden bewirkt zwar keine Schädigung, verbessert aber nur unwesentlich die Reinheit.

Der Extrakt wird mit Wasser (VE-Wasser), vorzugsweise 60 - 80 Vol.%, insbesondere bevorzugt 60-70 Vol. % der Extraktmenge, verdünnt und das Methylacetat unter Normaldruck abdestilliert. Dabei wird vorzugsweise eine Sumpftemperatur von maximal 70°C gewählt.

Der Extrakt sollte möglichst umgehend innerhalb 1 Stunde weiterverarbeitet werden , da sonst Reinheitseinbußen durch unkontrollierte Kristallisation auftreten können.

Die Zugabe der Wassermenge kann optional auch erst während der Destillation portionsweise erfolgen.

Das Destillat enthält noch ca. 5 Gew.% Wasser (nach Karl-Fischer) und kann nach Einstellung des Wassergehaltes wiederverwendet werden. Ca. 85 Gew.% des eingesetzten Methylacetats können so wiedergewonnen werden.

Der wäßrige Destillationsrückstand wird zügig, vorzugsweise innerhalb von maximal 2 Stunden, auf 10°C abgekühlt, wobei das Isoquercetin auszufallen beginnt. Unmittelbar bei dem Abkühlen fallen etwa 80% des insgesamt ausfallenden Isoquercetins sehr feinkristallin mit hoher Reinheit aus. Die Überprüfung per HPLC zeigt dabei, dass das Produkt aus 94 % Isoquercetin, 1 % Rutin und 0,2 % Quercetin besteht. Damit ist das bevorzugte Ziel, Isoquercetin mit einer Reinheit von über 90 % zu gewinnen, erreicht.

Das Produkt muss umgehend abgetrennt werden, da sich bei weiterem Rühren die Kristallstruktur vergröbert und der Gehalt sinkt, da im Wesentlichen nur noch Quercetin ausfällt.

Das Produkt wird dann getrocknet, was vorzugsweise im Vakuumschrank bei ca. 80 bis 120 mbar und einer Trocknungstemperatur von maximal 45°C passiert.

Sollte es einmal nicht möglich sein, den Extrakt umgehend weiterzuverarbeiten oder sollte das ausgefallenen Produkt versehentlich zu lange gestanden haben, ist mit Reinheitseinbußen zu rechnen. Die Reinheit des gewünschten Isoquercetins kann dann aber durch weitere Aufreinigungsschritte noch verbessert werden.

Als möglicher Aufreinigungsschritt kann beispielsweise das Ausrühren von Quercetinverunreinigungen mit Methyl-tert.butyl-ether genannt werden.

Eine andere Möglichkeit der Aufreinigung ist das kurze Aufkochen mit Wasser und anschließender Filtration.

Für die Durchführung in größerem Maßstab kann das erfindungsgemäße Verfahren auch halbkontinuierlich durchgeführt werden. Das halbkontinuierliche Verfahren ist auch Gegenstand dieser Erfindung.

Man verfährt dabei so, dass man die notwendige Lösungsmittelmenge (wobei das Lösungsmittelgemisch ebenfalls zusammengesetzt ist aus Methylacetat und Wasser im Volumenverhältnis 9:1) durch einen Extraktor mit Filterboden, der eine hochkonzentrierte Suspension von Mutteriaugenrückständen enthält, führt.

Extraktorvolumen und Volumenströme sind so bemessen, dass eine Verweilzeit im Extraktor von mindestens 30 Minuten eingehalten wird. Nach einer Einlaufphase von einer Verweilzeit, während der der Extraktor im Umlauf betrieben wird, führt man frisches, temperiertes Lösungsmittelgemisch in den Extraktor und zieht gleichzeitig dieselbe Menge Extrakt ab, so dass das Arbeitsvolumen im Extraktor konstant bleibt.
Die Temperatur im Extraktor wird dabei zwischen 35° und 50°C gewählt.

Der abgezogene Extrakt wird sofort bis zum wässrigen Rückstand eingeengt, wobei das Destillat (Wassergehalt 4,2%) nach Wassereinstellung direkt wieder zur Extraktion verwendet werden kann.

Werden die weiteren Schritte Kristallisation, gegebenenfalls auch das Auskochen mit Wasser und Ausrühren mit Methyl-tert.butyl-ether in derselben Apparatur nach Austrag des Extraktionsrückstandes ausgeführt, verbleibt die durchgängig feste Produktphase die ganze Zeit in der Apparatur, so dass einige Filtrationsschritte entfallen, wodurch Produkt- und Betriebsmittelverluste minimiert werden.

Bei der Aufreinigung des Rohisoquercetins kann das Auskochen mit Wasser auch vor die Quercetinentfernung mit MTBE-Ether gezogen werden, da ein Teil des Quercetins auch schon mit der wässrigen Mutterlauge entfernt werden kann.

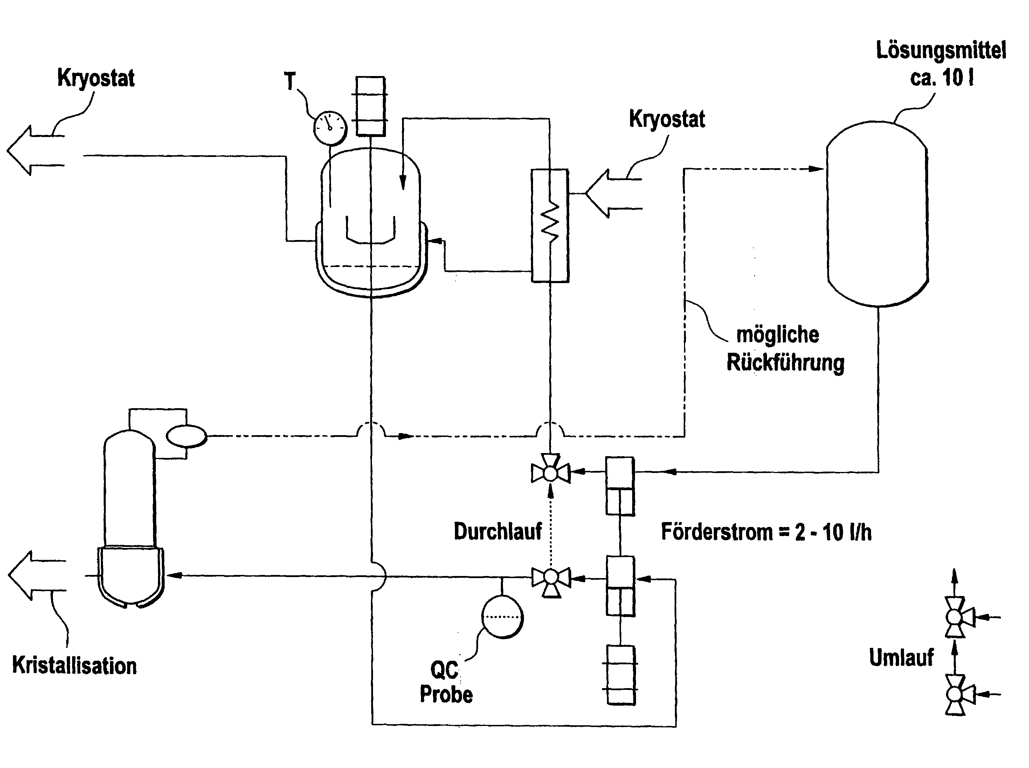

In Abbildung 1 ist ein Schema einer solchen halbkontinuierlichen Anlage beispielhaft aufgezeichnet.

Das erfindungsgemäße Verfahren im Batch-Verfahren ist jedoch, bei Durchführung in entsprechend großen Extraktionsgefäßen, vorzugsweise Email-Apparaturen, ebenfalls hervorragend geeignet, um große Mengen an Isoquercetin zu isolieren, und zwar in sehr reinem Zustand ohne weitere Reinigungsschritte.

Mit dem hier beschriebenen Verfahren ist nun eine Methode zugänglich geworden, hochreines Isoquercetin in größeren Mengen äußerst wirtschaftlich zu erhalten.

Auch ohne weitere Ausführungen wird davon ausgegangen, dass ein Fachmann die obige Beschreibung in weitesten Umfang nutzen kann. Die bevorzugten Ausführungsformen sind deswegen lediglich als beschreibende, keineswegs als in irgendeiner Weise limitierende Offenbarung aufzufassen.

Die vollständige Offenbarung aller vor- und nachstehend aufgeführten Anmeldungen und Veröffentlichungen sind durch Bezugnahme in diese Anmeldung eingeführt.

Die folgenden Beispiele werden die Erfindung noch erläutern.

### Beispiel 1

0,625 kg Ammoniumhydrogencarbonat werden in einem Anschlämmgefäß in 51 VE-Wasser vollständig aufgelöst. Man gibt 45 kg VE-Wasser in die Reaktionsapparatur (Email-Apparatur). 50 kg Pasta Seca werden über ein Alexander-Sieb in einen Zwischenbehälter gesiebt.

418 kg Methylacetat werden in die Reaktionsapparatur überführt und dann unter Rühren die gesiebte Pasta Seca langsam zugegeben.

Man extrahiert nun bei einer Innentemperatur von 45°C ca. 45 Minuten, überführt (Stickstoff) das Reaktionsgemisch in eine Zentrifuge und schleudert das Raffinat bei 300 U/min. ab. Anschließend wird der Rückstand trockengeschleudert bei 500 - 800 U/min (Schleudertuch Diolen). Das Raffinat läßt sich, aufgefüttert mit frischer Pasta Seca, zu weiteren Extraktionen einsetzen.

Der Extrakt wird nun mit 200 kg Wasser versetzt und das Methylacetat unter Normaldruck bei maximal 65°C Sumpftemperatur abdestilliert. Das Destillat enthält noch ca. 5 Gew.% Wasser (nach Karl-Fischer) und kann nach Einstellung des Wassergehaltes wiederverwendet werden. Ca. 85 Gew.% des eingesetzen Methylacetats werden so wiedergewonnen.

Der wäßrigen Destillationsrückstand wird innerhalb von 2h auf 10°C abgekühlt, wobei das Isoquercetin auszufallen beginnt. Unmittelbar bei dem Abkühlen fallen etwa 80% des insgesamt ausfallenden Isoquercetins sehr feinkristallin mit hoher Reinheit (HPLC: ca. 94 % Isoquercetin, 1 % Rutin, 0,2 % Quercetin) aus. Das Produkt muß umgehend abgetrennt werden (Zentrifuge), da sich bei weiterem Rühren die Kristallstruktur vergröbert und der Gehalt sinkt, da im Wesentlichen nur noch Quercetin ausfällt.

Der Filterkuchen wird mit Wasser gewaschen und trockengeschleudert.

Das Produkt wird bei einer Trocknungstemperatur von max. 45 °C und bei einem Druck im Vakuumschrank von ca. 100 mbar ± 20 mbar getrocknet. Man erhält so 3,49 kg trockenes Produkt.

### Beispiel 2

Im diesem Beispiel wird das halbkontinuierliche Verfahren im Labormaßstab beschrieben:
Ausgansmaterialien: 500 g Mutterlaugenrückstände (Pasta), 8370 g Methylacetat, VE-Wasser

Das Methylacetat (≅ 9000 ml) werden mit 1000 g VE-Wasser in einer Vorlage zum Extraktionsmittel vereinigt. 2 Liter dieses Lösungsmittelgemisches werden im Extraktor vorgelegt und auf 45°C temperiert.

500 g Mutterlaugenrückstände werden in den Extraktor eingerührt. Der Extrakt wird durch den Siebboden mit einem Volumenstrom von 4l/h abgesaugt und 30 min. wieder in den Extraktor zurückgeführt.

Nach 30 Minuten wird der Extrakt kontinuierlich einer Destillationsapparatur zugeführt; der Extraktionsmittelverlust wird durch kontinuierliches Zuführen des restlichen, temperierten Extraktionsmittels ausgeglichen, sodass die 21 Arbeitsvolumen im Extraktor konstant bleiben.

Nachdem das vorgelegte Extraktionsmittel nach etwas 2 h aufgebraucht ist, wird der verbleibende Rückstand trocken gesaugt.

Aus dem Extrakt wird während der Extraktion das Methylacetat vollständig unter Normaldruck abdestilliert. Das Destillat enthält noch 4,2 % Wasser (nach Karl-Fischer) und kann nach Einstellung des Wassergehalts erneut als Extraktionsmittel eingesetzt werden.

Den verbleibenden, wässrigen Extraktionsrückstand lässt man unter Rühren auf Raumtemperatur abkühlen, saugt ihn trocken und wäscht mit wenig Extraktionsmittel nach. Man erhält das Rohisoquercetin TS (Trokkensubstanz) = 135,4 g , HPLC: Isoquercetin: 70 %, Rutin: 25 %, Quercetin: 1 %, Rest: 4%.

100 g des Rohisoquercetins werden in 1000 g VE-Wasser suspendiert und 1 Min. am Siedern gehalten und sofort abilfitriert. Man erhält so reines Isoquercetin (HPLC: 90%) in einer Menge von 44 g.

Aus dem Extrakt kristallisiert Isoquercetin, das sich zusammen mit Rohisoquercetin in der Aufreinigung wieder einsetzen lässt.

## Patentansprüche

1. Verfahren zur Gewinnung von Isoquercetin aus Bioflavanoidpasten (Mutterlaugenrückstände), **dadurch gekennzeichnet, dass** die Mutterlaugenrückstände (Pasten) mit einem Lösungsmittelgemisch aus Methylacetat und Wasser extrahiert werden.

2. Verfahren nach Anspruch 1, **dadurch gekennzeichnet, dass** folgende Schritte durchgeführt werden
a) Extrahieren der Mutterlaugenrückstände (Pasten) mit einem Lösungsmittelgemisch aus Methylacetat und Wasser im Volumenverhältnis 9:1 in zehnfachem Überschuss, bezogen auf die Masse der eingesetzten Mutterlaugenrückstände, und zwar bei einer Extraktionstemperatur von 35° bis 50°C, und einer Extraktionszeit von mindestens 30 Minuten, und Abschleudern vom festen Raffinat,
b) Verdünnen des Extraktes mit Wasser,
c)Abdestillieren des Methylacetats aus dem Extrakt unter Normaldruck,
d) rasches Abkühlen des wässrigen Destillationsrückstandes auf 10°C, wobei das Isoquercetin auszufallen beginnt,
e) umgehendes Abtrennen und Trocknen des Produktes.

3. Verfahren nach Anspruch 1 oder 2, **dadurch gekennzeichnet, dass** als Bioflavanoidpaste die Mutterlaugenrückstände verwendet werden, die bei der Rutinherstellung aus Dimorphandra Gardneriana anfallen.

4. Verfahren nach einem der Ansprüche 1 bis 3, **dadurch gekennzeichnet, dass** das Lösungsmittelgemisch mit NH₄HCO₃ gepuffert ist.

5. Verfahren nach einem der Ansprüche 1 bis 4, **dadurch gekennzeichnet, dass** die Extraktionszeit 30 bis 60 Minuten dauert.

6. Verfahren nach einem der Ansprüche 1 bis 5, **dadurch gekennzeichnet, dass** im Schritt b) mit ca. 60 - 80 Vol% Wasser, bezogen auf die Extraktmenge, verdünnt wird.

7. Verfahren nach einem der Ansprüche 1 bis 6, **dadurch gekennzeichnet, dass** die Zugabe von Wasser (Schritt b) auch während der Destillation (Schritt c) erfolgen kann.

8. Verfahren nach einem der Ansprüche 1 bis 7, **dadurch gekennzeichnet, dass** das rasche Abkühlen des wässrigen Destillationsrückstandes innerhalb 2 Stunden erfolgt.

9. Verfahren nach Anspruch 1 oder 2, **dadurch gekennzeichnet, dass** es als halbkontinuierliches Verfahren durchgeführt wird.

10. Verfahren nach Anspruch 9, **dadurch gekennzeichnet, dass** man die notwendige Lösungsmittelmenge durch einen Extraktor mit Filterboden, der eine hochkonzentrierte Suspension von Mutterlaugenrückständen enthält, führt,
dass Extraktorvolumen und Volumenströme so bemessen sind, dass eine Verweilzeit im Extraktor von mindestens 30 Minuten eingehalten wird,
dass die Temperatur im Extraktor zwischen 35° und 50°C gehalten wird,
dass man frisches, temperiertes Lösungsmittelgemisch in den Extraktor führt und gleichzeitig dieselbe Menge Extrakt abzieht, so dass das Arbeitsvolumen im Extraktor konstant bleibt,
dass der abgezogene Extrakt sofort bis zum wässrigen Rückstand eingeengt wird,
dass gegebenenfalls die weiteren Schritte Kristallisation, Auskochen mit Wasser und Ausrühren mit Methyl-tert.butylether in derselben Apparatur nach Austrag des Extraktionsrückstandes ausgeführt werden.

## Claims

1. Process for the isolation of isoquercetin from bioflavonoid pastes (mother liquor residues), **characterised in that** the mother liquor residues (pastes) are extracted with a solvent mixture comprising methyl acetate and water.

2. Process according to Claim 1, **characterised in that** the following steps are carried out:
a) extraction of the mother liquor residues (pastes) with a solvent mixture comprising methyl acetate and water in the volume ratio 9:1 in a ten-fold excess, based on the weight of the mother liquor residues employed, at an extraction temperature of from 35° to 50°C and an extraction time of least 30 minutes, and separation from the solid raffinate by centrifugation,
b) dilution of the extract with water,
c) removal of the methyl acetate from the extract by distillation under atmospheric pressure,
d) rapid cooling of the aqueous distillation residue to 10°C, during which the isoquercetin starts to precipitate,
e) immediate separation and drying of the product.

3. Process according to Claim 1 or 2, **characterised in that** the bioflavonoid paste used comprises the mother liquor residues formed in the preparation of rutin from Dimorphanda gardneriana.

4. Process according to one of Claims 1 to 3, characterised that the solvent mixture is buffered with NH₄HCO₃.

5. Process according to one of Claims 1 to 4, **characterised in that** the extraction time has a duration of from 30 to 60 minutes.

6. Process according to one of Claims 1 to 5, **characterised in that** the dilution in step b) is carried out with approximately 60 - 80% by volume of water, based on the amount of extract.

7. Process according to one of Claims 1 to 6, **characterised in that** the addition of water (step b) can also take place during the distillation (step c).

8. Process according to one of Claims 1 to 7, **characterised in that** the rapid cooling of the aqueous distillation residue takes place within 2 hours.

9. Process according to Claim 1 or 2, **characterised in that** it is carried out as a semi-continuous process.

10. Process according to Claim 9, **characterised in that** the requisite amount of solvent is passed through an extractor with filter base which contains a highly concentrated suspension of mother liquor residues,
the extractor volume and volume flow rates are dimensioned in such a way that a residence time in the extractor of at least 30 minutes is maintained,
the temperature in the extractor is held between 35° and 50°C,
fresh, temperature-controlled solvent mixture is passed into the extractor and at the same time the same amount of extract is withdrawn, so that the working volume in the extractor remains constant,
the withdrawn extract is immediately concentrated to give the aqueous residue,
if desired, the further steps of crystallisation, boiling with water and stirring with methyl tert-butyl ether are carried out in the same apparatus after discharge of the extraction residue.

## Revendications

1. Procédé d'isolement de l'isoquercétine depuis des pâtes de bioflavonoïdes (résidus de liqueur mère), **caractérisé en ce que** les résidus de liqueur mère (pâtes) sont extraits par un mélange de solvants comprenant de l'acétate de méthyle et de l'eau.

2. Procédé selon la revendication 1, **caractérisé en ce que** l'on réalise les étapes suivantes :
a) l'extraction des résidus de liqueur mère (pâtes) par un mélange de solvants comprenant de l'acétate de méthyle et de l'eau dans un rapport volumique de 9:1 et de dix fois en excès, sur la base du poids des résidus de liqueur mère employés, à une température d'extraction allant de 35° à 50°C et sur une période d'extraction d'au moins 30 minutes, et la séparation du produit raffiné solide par centrifugation,
b) la dilution de l'extrait par l'eau,
c) l'élimination de l'acétate de méthyle depuis l'extrait par distillation sous pression atmosphérique,
d) le refroidissement rapide du résidu de distillation aqueux jusqu'à 10°C, pendant lequel l'isoquercétine commence à précipiter,
e) la séparation immédiate et le séchage du produit.

3. Procédé selon la revendication 1 ou 2, **caractérisé en ce que** la pâte de bioflavonoïdes utilisée comprend les résidus de liqueur mère formés lors de la préparation de rutine à partir de Dimorphanda gardneriana.

4. Procédé selon l'une des revendications 1 à 3, **caractérisé en ce que** le mélange de solvants est tamponné par NH₄HCO₃.

5. Procédé selon l'une des revendications 1 à 4, **caractérisé en ce que** la période d'extraction a une durée allant de 30 à 60 minutes.

6. Procédé selon l'une des revendications 1 à 5, **caractérisé en ce que** la dilution à l'étape b) est réalisée par environ 60 - 80% en volume d'eau, sur la base de la quantité d'extrait.

7. Procédé selon l'une des revendications 1 à 6, **caractérisé en ce que** l'addition d'eau (étape b) peut également avoir lieu lors de la distillation (étape c).

8. Procédé selon l'une des revendications 1 à 7, **caractérisé en ce que** le refroidissement rapide du résidu de distillation aqueux a lieu dans les 2 heures.

9. Procédé selon la revendication 1 ou 2, **caractérisé en ce qu'**il est réalisé en tant que procédé semi-continu.

10. Procédé selon la revendication 9, **caractérisé en ce que** la quantité de solvant requise est passée à travers un extracteur ayant une base filtrante contenant une suspension hautement concentrée en résidus de liqueur mère,
le volume de l'extracteur et les débits volumiques sont dimensionnés tels qu'un temps de séjour dans l'extracteur d'au moins 30 minutes est maintenu,
la température dans l'extracteur est maintenue entre 35° et 50°C,
un mélange de solvants frais, à température contrôlée est passé dans l'extracteur et en même temps la même quantité d'extrait est retirée, de sorte que le volume utile dans l'extracteur reste constant,
l'extrait retiré est immédiatement concentré pour donner le résidu aqueux,
si on le désire, les étapes supplémentaires de cristallisation, d'ébullition par de l'eau et d'agitation avec du méthyltertio-butyléther sont réalisées dans le même appareil après la décharge du résidu d'extraction.
